# EUROPÄISCHE PATENTSCHRIFT

(11) **EP 0 374 464 B1**
(45) Veröffentlichungstag und Bekanntmachung des Hinweises auf die Patenterteilung: **14.06.1995**
(21) Anmeldenummer: 89120924.9
(22) Anmeldetag: 11.11.1989
(51) Int. Cl.: A61B 17/12

(54) **Venenstauvorrichtung für Körperteile**
Occluding device for parts of the human body
Dispositif d'occlusion veineuse pour parties du corps humain

(30) Priorität: 25.11.1988 DE 3839794
(43) Veröffentlichungstag der Anmeldung: 27.06.1990
(73) Patentinhaber: PRÄMETA Gesellschaft für Präzisionsmetall- und Kunststofferzeugnisse mbH & Co. KG, 51107 Köln (DE)
(72) Erfinder: Wehking, Wolfgang, D-5000 Köln (DE)
(74) Vertreter: Dallmeyer, Georg, Dipl.-Ing.

(56) Entgegenhaltungen:
- DE-A- 3 602 778
- DE-U- 8 409 491
- DE-U- 8 436 766

## Beschreibung

Die Erfindung bezieht sich auf eine Venenstauvorrichtung für Körperteile mit einem Schloßgehäuse und mit einem in dem Schloßgehäuse fest integrierten, schwenkbar gelagerten Klemmhebel für das durch das Schloßgehäuse hindurchgeführte Band, wobei das andere Bandende mittels eines Halteorgans am Klemmhebel einrastbar oder durch das Schloßgehäuse ebenfalls hindurchführbar ist.

Die bekannten Venenstauvorrichtungen bzw. Abschnürvorrichtungen für Körperteile sind üblich so ausgebildet, daß zur schwenkbaren Lagerung des Klemmhebels ein Achsstift vorgesehen ist, der beiderseits in den Seitenwangen des Schloßgehäuses gelagert ist. Hierbei weisen die Seitenwangen Bohrungen auf, die bis zur Außenfläche der Seitenwangen führen, so daß der Achsstift von außen quer durch das Schloßgehäuse hindurchgeführt werden kann. Die äußeren Flächen der Seitenwangen des Schloßgehäuses wurden in der Weise bearbeitet, daß die Stirnflächen des Achsstiftes mit den Außenflächen der Seitenwangen bündig verlaufen. Der Achsstift ist von außen zwar sichtbar, aber die Außenflächen der Seitenwangen bleiben glatt. Es hat sich herausgestellt, daß bei vielem Gebrauch der Abschnürvorrichtung der Achsstift nicht immer in seiner Lage verbleibt, sondern sich nach der einen oder der anderen Richtung verschiebt, so daß er an den Außenflächen der Seitenwangen entweder vorsteht oder zurücksteht. Beides ist bei der Handhabung im ärztlichen Bereich unbefriedigend. In dem einen Fall bietet der vorstehende Achsstift die Möglichkeit einer leichten Verletzung der Haut der Bedienungsperson. Das Zurückstehen des Achsstiftes führt zu einer Ausnehmung, in der sich Bakterien o.dgl. festsetzen können.

Es ist grundsätzlich zu unterscheiden zwischen Venenstauvorrichtungen mit fest integriertem schwenkbar gelagerten Klemmhebel und solchen Venenstauvorrichtungen, die ein zweiteiliges, parallel zum durchgeführten Band teilbares Gehäuse aufweisen, dessen Oberteil zugleich einen herausnehmbaren Klemmhebel bildet. Bei diesen Ausführungen z.B. nach DE-U-84 36 766 besteht die Venenstauvorrichtung aus einem im Querschnitt im wesentlichen U-förmigen Gehäuse und einem Gehäuseoberteil, das gleichzeitig den Klemmhebel bildet, wobei das Oberteil in das U-förmige Gehäuseteil einlegbar ist. Zum Lösen einer derartigen Venenstauvorrichtung wird das Oberteil nach Verschiebung aus der Raststellung von dem Unterteil nach oben abgenommen. Bei derartigen Venenstauvorrichtungen kann das der Erfindung zugrundeliegende Problem gar nicht entstehen, da der Klemmhebel nicht fest in dem Schloßgehäuse integriert ist.

Eine bekannte Venenstauvorrichtung (DE-A-36 02 778) weist ein Schloßgehäuse auf mit einem in dem Schloßgehäuse unlösbar integrierten, schwenkbar gelagerten Klemmhebel für das durch das Schloßgehäuse hindurchgeführte Ende eines Bandes. Das andere Bandende ist mittels eines Halteorgans am Gehäuse einrastbar.

Eine weitere gattungsgemäße Venenstauvorrichtung (DE 28 24 037) beschreibt ein Schloßgehäuse mit fest integriertem, schwenkbar gelagerten Klemmhebel für das durch das Schloßgehäuse hindurchgeführte Ende eines Bandes, wobei das andere Bandende mittels eines Halteorgans am Klemmhebel einrastbar ist, so daß die Bandschlaufenspannung auf den Klemmhebel übertragen wird.

Bei derartigen Venenstauvorrichtungen mit unlösbar integriertem Klemmhebel besteht das Problem, daß der Klemmhebel mit einem Achsstift in dem Schloßgehäuse gelagert ist, wobei der Achsstift seinerseits in den Seitenwangen des Schloßgehäuses gelagert ist. Die Seitenwangen des Schloßgehäuses sind zwecks Aufnahme des Achsstiftes mit Bohrungen versehen, die bis zur Außenfläche der Seitenwangen führen. In diese Bohrung kann der Achsstift von außen quer durch das Schloßgehäuse hindurchgeführt werden. Wie bereits erläutert, ist eine derartige Lagerung eines fest mit dem Schloßgehäuse verbundenen Klemmhebels unhygienisch. Des weiteren besteht eine Verletzungsgefahr, wenn der Achsstift über die Seitenwangen des Schloßgehäuses übersteht.

Der Erfindung liegt die Aufgabe zugrunde, bei einer Venenstauvorrichtung mit in dem Schloßgehäuse fest integriertem Klemmhebel die Verletzungsgefahr zu verringern und die hygienischen Bedingungen zu verbessern.

Zur Lösung dieser Aufgabe dienen die Merkmale des Anspruchs 1.

Durch eine solche Ausbildung der Lagerung des Klemmhebels wird in einfacher und zuverlässiger Weise verhindert, daß ein Entstehen von vorstehenden Teilen oder zurückliegenden Ausnehmungen auftreten kann. Die Außenflächen der Seitenwangen sind durchweg geschlossene Flächen, die keinen Grund mehr zur Beeinträchtigung bei der Handhabung der Abschnürvorrichtung abgeben können. Die Außenflächen der Seitenwangen können durchweg glatt und damit in hygienischer Hinsicht leicht sauber gehalten werden. Dabei leidet der Lagermechanismus für die Drehachse des Klemmhebels in keiner Weise. Er kann sogar stärker ausgebildet werden als bisher.

Vorteilhaft weist der Klemmhebel an den Seitenflächen vorstehende Achsstutzen auf, die in Sackbohrungen der Gehäuse-Seitenwangen eingreifen. Dabei können die Achsstutzen mit festem Sitz in einer Bohrung des Klemmhebels eingepreßt sein. Die Achsstutzen können an dem Klemmhebel eingesetzt, aber auch direkt angeformt sein.

Gemäß einem weiteren Merkmal der Erfindung können in den Innenflächen der Gehäuse-Seitenwangen oder an den seitlichen Außenflächen des Klemmhebels Nuten vorgesehen sein, in die die Achsstutzen für den Klemmhebel eingreifen. Zur Festlegung der Achse oder der Achsstutzen können die Nuten mittels Füllstücke o.dgl. teilweise oder auf ihrer gesamten Länge verschlossen werden.

Die zur Aufnahme der Achsstutzen dienenden Nuten können längs oder quer zu den Gehäuse-Seitenwangen verlaufen.

Es ist zweckmäßig, daß die Nuten mit Hinterschneidungen versehen werden, wobei die Füllstücke im Querschnitt der hinterschnittenen Nut angepaßt sind. Die Füllstücke können durch Preßsitz in den Nuten festsitzen. Das Festsetzen der Füllstücke in den Nuten kann ferner mittels Sperrzinken o.dgl. oder mittels Kerben o.dgl. erreicht werden.

Die Erfindung wird anhand der in der Zeichnung dargestellten Beispiele erläutert.
- Fig. 1: zeigt eine Ausführungsform einer Venenstauvorrichtung gemäß der Erfindung im Längsschnitt und im Schema.
- Fig. 2: stellt eine Stirnansicht auf das Schloßgehäuse schematisch dar.
- Fig. 3 und 4: veranschaulichen zwei Ausführungsformen der Achslagerung des Klemmhebels in Seitenansicht schematisch.
- Fig. 5: zeigt das Schloßgehäuse für sich im Längsschnitt mit einer Längsnut zur Aufnahme des Achsstutzens.
- Fig. 6 und 7: stellen verschiedene Ausführungsformen für die Ausbildung des Füllstückes der den Achsstutzen aufnehmen Nuten schematisch dar.
- Fig. 8: zeigt einen Längsschlitz durch das Schloßgehäuse mit senkrecht verlaufender Nut zur Aufnahme des Achsstutzens.
- Fig. 9: ist eine Stirnansicht der Ausführungsform der Fig. 8, im Querschnitt gezeichnet.

Die Venenstauvorrichtung 1 bzw. die Abschnürvorrichtung für Körperteile weist ein Schloßgehäuse 2 auf, in dem ein Klemmhebel 3 um eine Achse 4 schwenkbar gelagert ist. In dem Klemmhebel 3 kann ein Halteorgan 5 schnappend eingerastet werden, an dem das Bandende 6 des Bandes 7 befestigt ist, wobei die Einraststellung des Halteorganes 5 durch eine Feder 8 unterstützt wird. Das andere Bandende 9 ist durch das Schloßgehäuse 2 hindurchgeführt.

Die Achse 4 für den Klemmhebel 3 ist in bezug auf das Schloßgehäuse 2 so ausgebildet, daß sie vor den Außenflächen der Gehäuse-Seitenwangen 2a, 2b endet, so daß die Außenflächen der Seitenwangen massiv geschlossen ausgebildet sind.

Der Klemmhebel 3 weist bevorzugt an den Seitenflächen vorstehende Achsstutzen 11 und 12 auf, die in sackförmige Ausnehmungen, z.B. Bohrungen der Gehäuse-Seitenwangen 2a und 2b eingreifen. Die Achsstutzen 11 und 12 können in einer Bohrung des Klemmhebels 3 eingepreßt sein. Sie können aber auch an dem Klemmhebel 3 unmittelbar angeformt sein. Man kann aber auch einen durchgehenden Achsstift 15 bei dem Klemmhebel 3 vorsehen, der an den Seitenflächen nur soweit vorragt, daß der Stift in den Seitenwangen 2a und 2b nicht nach außen tritt.

Die Ausnehmungen 13 und 14, die die Achsstutzen 11 und 12 aufnehmen, sind zweckmäßig als Nuten ausgebildet, wobei die Nuten durch Füllstücke teilweise oder auf ihrer gesamten Länge verschlossen gehalten werden, nachdem die Achsstutzen in den Nuten Aufnahme gefunden haben.

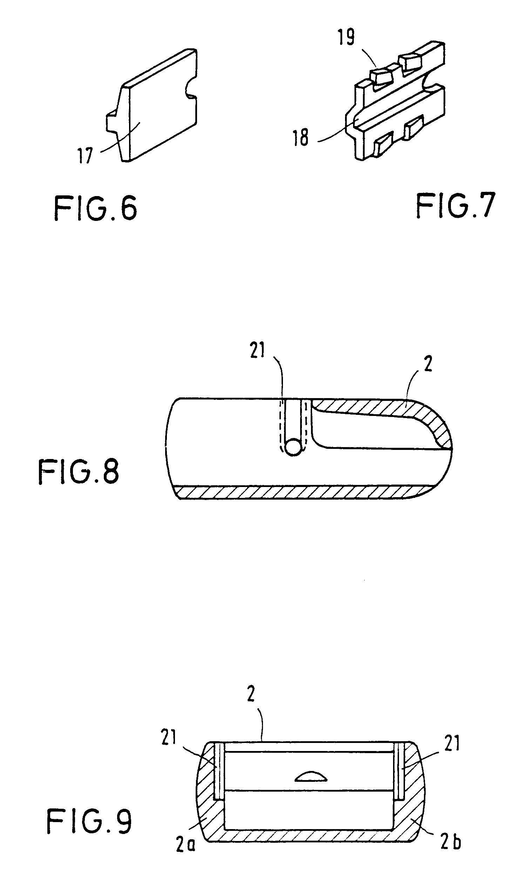

Die Nuten 13, 14 sind vorteilhaft als hinterschnittene Nuten ausgebildet, wobei die Füllstücke dem Querschnitt der Nut mit Hinterschneidung angepaßt sind. In den Figuren 6 und 7 sind zwei Ausführungsformen der Füllstücke schaubildlich dargestellt. Das Füllstück 17 kann durch Preßsitz in den Nuten unverrückbar gehalten werden. Man kann aber auch ein Füllstück 18 verwenden, das mit Sperrzinken oder Sperrlappen 19 versehen ist, die ein Festsitzen des Füllstückes in den Nuten 13, 14 veranlassen.

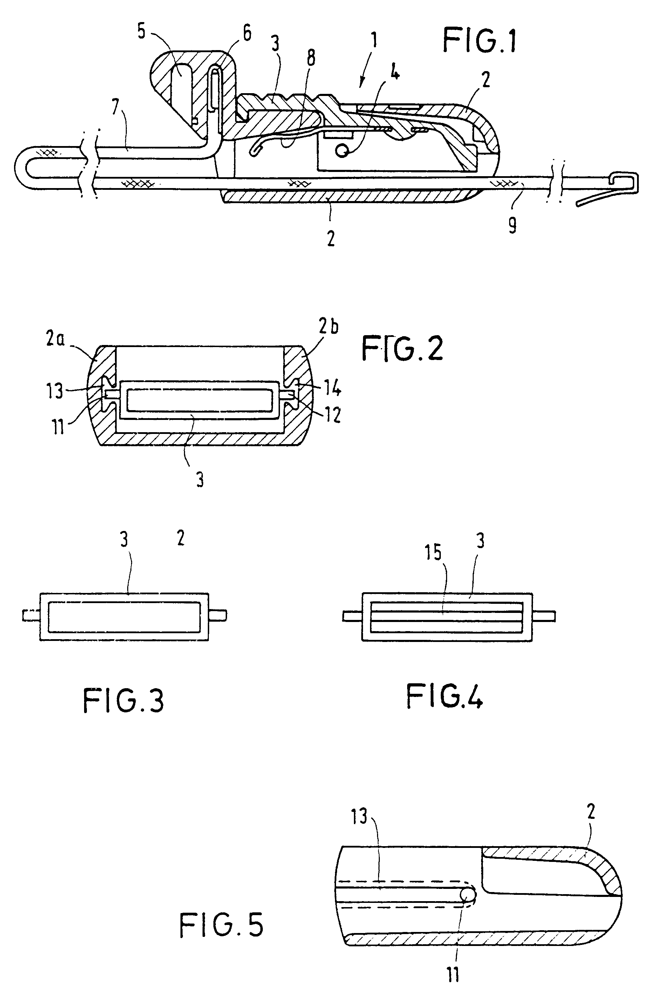

Die Nuten, in denen die Achsstutzen Aufnahme finden, können in Richtung der Längsachse des Schloßgehäuses 2 vorgesehen sein, wie dies aus Figur 5 zu entnehmen ist. Man kann aber auch Nuten 21 an dem Schloßgehäuse anbringen, die senkrecht zur Längsachse desselben verlaufen (Fig. 8,9). Hierbei wird der Klemmhebel mit seinen Achsstutzen von oben in die Nuten 21 eingesetzt. Ein entsprechend passendes Füllstück verschließt die Nuten 21 von oben.

Es ist ferner möglich, daß die Seitenwangen des Schloßgehäuses an ihrer Innenseite mit festsitzenden Achsstutzen versehen sind, die in entsprechend geführten Nuten des Klemmhebels eingreifen, wobei das Verschließen der Nuten durch passende Füllstücke erfolgen kann.

## Patentansprüche

1. Venenstauvorrichtung (1) für Körperteile, mit einem Schloßgehäuse (2) und mit einem in dem Schloßgehäuse (2) fest integrierten, schwenkbar gelagerten Klemmhebel (3) für das durch das Schloßgehäuse (2) hindurchgeführte Ende eines Bandes, wobei das andere Bandende (6) mittels eines Halteorganes (5) am Klemmhebel (3) eingerastet oder durch das Schloßgehäuse (2) ebenfalls hindurchgeführt ist,
**dadurch gekennzeichnet**,
daß die Achse (4) für den in das Schloßgehäuse (2) fest integrierten Klemmhebel (3) in Nuten (13, 14; 21) in den Innenflächen der Gehäuse-Seitenwangen (2a, 2b) oder an den seitlichen Außenflächen des Klemmhebels (3) eingreift, daß die Nuten (13, 14; 21) mittels Füllelementen (17, 18) teilweise oder auf ihrer gesamten Länge verschließbar sind und daß die Außenflächen der Gehäuse-Seitenwangen (2a, 2b) geschlossen, d.h. augenfrei ausgebildet sind.

2. Venenstauvorrichtung nach Anspruch 1, dadurch gekennzeichnet, daß der Klemmhebel (3) an den Seitenflächen vorstehende Achsstutzen (11,12) aufweist, die in Sackausnehmungen (13,14) der Gehäuse-Seitenwangen (2a,2b) eingreifen.

3. Venenstauvorrichtung nach einem der Ansprüche 1 oder 2, dadurch gekennzeichnet, daß die Nuten (13,14) in den Gehäuse-Seitenwangen (2a,2b) längs verlaufen.

4. Venenstauvorrichtung nach einem der Ansprüche 1 oder 2, dadurch gekennzeichnet, daß die Nuten (21) in den Gehäuse-Seitenwangen (2a,2b) senkrecht verlaufen.

5. Venenstauvorrichtung nach einem der Ansprüche 1 bis 4, dadurch gekennzeichnet, daß die Nuten (13,14;21) Hinterschneidungen aufweisen, und daß die Füllstücke (17,18) dem Querschnitt der Nuten mit Hinterschneidung angepaßt sind.

6. Venenstauvorrichtung nach einem der Ansprüche 1 bis 5, dadurch gekennzeichnet, daß die Füllstücke (17) durch Preßsitz in den Nuten (13,14;21) festsitzen.

7. Venenstauvorrichtung nach einem der Ansprüche 1 bis 5, dadurch gekennzeichnet, daß die Füllstücke (18) durch Sperrzinken oder Sperrlappen (19) in der Nut festgelegt sind.

8. Venenstauvorrichtung nach einem der Ansprüche 2 bis 7, dadurch gekennzeichnet, daß die Achsstutzen (11,12) in einer Bohrung des Klemmhebels (3) eingepreßt sind.

9. Venenstauvorrichtung nach einem der Ansprüche 2 bis 7, dadurch gekennzeichnet, daß die Achsstutzen (11,12) an dem Klemmhebel (3) angeformt sind.

10. Venenstauvorrichtung nach einem der Ansprüche 2 bis 7, dadurch gekennzeichnet, daß die Achsstutzen (11,12) Teil eines durch den Klemmhebel (3) hindurchgeführten Achsstiftes bilden.

11. Venenstauvorrichtung nach einem der Ansprüche 2 bis 7, dadurch gekennzeichnet, daß die Achsstutzen (11,12) an den Seitenwangen (2a,2b) des Schloßgehäuses (2) und die Nuten an dem Klemmhebel (3) angeordnet sind.

## Claims

1. A venous tourniquet device (1) for extremities, having a lock casing (2) and a clamping lever (3) for one end of a strap (7) passing through said lock casing (2), said lever being fixedly integrated and pivotally supported within the lock casing (2), the other end of the strap (7) being adapted to be engaged with the clamping lever (3) by means of a holding member (5) or being adapted for also being passed through the lock casing (2),
characterized in that
the axle (4) for the clamping lever (3) fixedly integrated in the lock casing (2) engages in grooves (13, 14; 21) in the inner surfaces of said lateral casing cheeks (2a, 2b) or the lateral outer faces of the clamping lever (3),
said grooves (13,14;21) are closed with filling members (17, 18) over parts or their entire length, and
the axle (4) for said clamping lever (3) ends before the outer surfaces of the lateral casing cheeks (2a, 2b) and that the outer surfaces of the cheeks are closed, i.e. without eyes.

2. The venous tourniquet device according to claim 1, characterized in that said clamping lever (3) has its lateral faces provided with projecting axle stubs (11,12) that engage in pocket bores (13, 14) of the lateral casing cheeks (2a, 2b).

3. The venous tourniquet device according to claim 1 or 2, characterized in that said grooves (13, 14) extend longitudinally in said lateral casing cheeks (2a, 2b).

4. The venous tourniquet device according to one of claims 1 to 3, characterized in that said grooves (21) extend vertically in said lateral casing cheeks (2a, 2b).

5. The venous tourniquet device according to one of claims 1 to 4, characterized in that said grooves (13,14;21) are provided with undercuts and that said filling members (17, 18) are adapted to the cross section of said grooves with undercut.

6. The venous tourniquet device according to one of claims 1 to 5, characterized in that said filling members (17) are fixed in said grooves (13, 14; 21) by tight fitting engagement.

7. The venous tourniquet device according to one of claims 1 to 5, characterized in that said filling members (18) are fixed in said grooves (13, 14; 21) by means of locking studs or locking lobes (19).

8. The venous tourniquet device according to one of claims 2 to 7, characterized in that said axle stubs (11,12) are pressed into a bore of said clamping lever (3).

9. The venous tourniquet device according to one of claims 2 to 7, characterized in that said axle stubs (11,12) are formed to said clamping lever (3).

10. The venous tourniquet device according to one of claims 2 to 7, characterized in that said axle stubs (11,12) form a part of an axle pin passed through said clamping lever (3).

11. The venous tourniquet device according to one of claims 2 to 7, characterized in that axle stubs (11,12) are disposed at the lateral cheeks (2a,2b) of the lock casing (2) and that the grooves are disposed at said clamping lever (3).

## Revendications

1. Dispositif d'occlusion veineuse (1) pour parties du corps, comportant un boîtier de fermeture (2) et un levier de serrage (3), incorporé en permanence dans le boîtier de fermeture et porté par lui avec liberté de pivotement, pour l'extrémité d'un lien guidée à travers le boîtier de fermeture (2), l'autre extrémité (6) du lien étant fixée par encliquetage au levier de serrage (3) au moyen d'un organe de maintien (5) ou étant également guidée à travers le boîtier de fermeture (2),
caractérisé
par le fait que l'axe (4) du levier de serrage (3) incorporé en permanence dans le boîtier de fermeture (2), s'ajuste dans des rainures (13, 14; 21) prévues dans les surfaces intérieures des joues latérales (2a, 2b) du boîtier ou sur les surfaces extérieures latérales du levier de serrage (3), que les rainures (13, 14; 21) peuvent être obturées, partiellement ou sur toute leur longueur, au moyen d'éléments de remplissage (17, 18) et que les surfaces extérieures des joues latérales (2a, 2b) du boîtier sont fermées, c'est-à-dire ne comportent pas de trou.

2. Dispositif d'occlusion veineuse selon la revendication 1, caractérisé par le fait que le levier de serrage (3) présente des tourillons (11, 12) qui dépassent sur les surfaces latérales et s'ajustent dans des évidements borgnes (13, 14) des joues latérales (2a, 2b) du boîtier.

3. Dispositif d'occlusion veineuse selon l'une des revendications 1 ou 2, caractérisé par le fait que les rainures (13, 14) prévues dans les joues latérales (2a, 2b) du boîtier sont orientées longitudinalement.

4. Dispositif d'occlusion veineuse selon l'une des revendications 1 à 3, caractérisé par le fait que les rainures (21) prévues dans les joues latérales (2a, 2b) du boîtier sont orientées perpendiculairement.

5. Dispositif d'occlusion veineuse selon l'une des revendications 1 à 4, caractérisé par le fait que les rainures (13, 14; 21) présentent des contre-dépouilles et que les éléments de remplissage (17, 18) sont adaptés à la section des rainures en contre-dépouille.

6. Dispositif d'occlusion veineuse selon l'une des revendications 1 à 5, caractérisé par le fait que les éléments de remplissage (17) sont fixés dans les rainures (13, 14; 21) par ajustement serré.

7. Dispositif d'occlusion veineuse selon l'une des revendications 1 à 5, caractérisé par le fait que les éléments de remplissage (8) sont fixés dans la rainure par des dents ou des languettes (19) de verrouillage.

8. Dispositif d'occlusion veineuse selon l'une des revendications 2 à 7, caractérisé par le fait que les tourillons (11, 12) sont montés, à ajustement serré, dans un perçage du levier de serrage (3).

9. Dispositif d'occlusion veineuse selon l'une des revendications 2 à 7, caractérisé par le fait que les tourillons (11, 12) sont formés sur le levier de serrage (3).

10. Dispositif d'occlusion veineuse selon l'une des revendications 2 à 7, caractérisé par le fait que les tourillons (11, 12) forment une partie d'un axe qui traverse le levier de serrage (3).

11. Dispositif d'occlusion veineuse selon l'une des revendications 2 à 7, caractérisé par le fait que les tourillons (11, 12) sont disposés sur les joues latérales (2a, 2b) du boîtier de fermeture (2) et que les rainures le sont sur le levier de serrage (3).
